Europäisches Patentamt

European Patent Office

Office européen des brevets

(19)

(11) Numéro de publication : **0 278 847 B1**

# FASCICULE DE BREVET EUROPEEN

(12)

(45) Date de publication du fascicule du brevet :
**15.01.92 Bulletin 92/03**

(51) Int. Cl.⁵ : **A61N 1/20**

(21) Numéro de dépôt : **88400214.8**

(22) Date de dépôt : **29.01.88**

(54) **Perfectionnements aux dispositifs pour l'excitation de la sécrétion salivaire.**

(30) Priorité : **29.01.87 FR 8701075**

(43) Date de publication de la demande :
**17.08.88 Bulletin 88/33**

(45) Mention de la délivrance du brevet :
**15.01.92 Bulletin 92/03**

(84) Etats contractants désignés :
**AT BE CH DE ES FR GB GR IT LI LU NL SE**

(56) Documents cités :
**EP-A- 0 145 673
FR-A- 2 439 592
FR-A- 2 546 070**

(73) Titulaire : **Jaffreo, Albert
2, rue Aimé Pinel
F-38230 Pont de Cheruy (FR)**

(72) Inventeur : **Jaffreo, Albert
2, rue Aimé Pinel
F-38230 Pont De Cheruy (FR)**
Inventeur : **Nouvel-Rousselot, Colette
91, avenue des Ternes
F-75017 Paris (FR)**

(74) Mandataire : **Wagret, Jean-Michel et al
CABINET WAGRET 23, rue de Léningrad
F-75008 Paris (FR)**

## Description

La présente invention concerne un dispositif en vue de compenser l'insuffisance de sécrétion salivaire en provoquant une excitation localisée propre à entraîner un accroissement dans la production des glandes salivaires.

L'insuffisance de sécrétion salivaire constitue une affection ou un disfonctionnement, qui pour être local et peu susceptible d'entraîner des troubles graves, n'en est pas moins particulièrement inconfortable et pénible pour le sujet.

Ce mauvais fonctionnement peut avoir des causes multiples, parfois permanentes et liées à une altération des tissus, par exemple à l'occasion et à la suite d'un traitement ayant engendré une nécrose, voire une détérioration des tissus pour lutter contre une tumeur maligne ; parfois cette insuffisance de sécrétion peut être momentanée et liée à un trouble émotionnel ou à la consommation de certains médicaments notamment des tranquilisants du type neuroleptique.

Les traitements connus actuellement visent essentiellement à pratiquer ou à remplacer une excitation de la glande salivaire par des moyens chimiques.

On a prévu, notamment aux termes du brevet français FR-A-2439592, un dispositif constitué d'un corps inerte apte à être mis en place dans la cavité buccale et produisant un courant d'énergie électrique par l'intermédiaire de deux pôles disposées de façon à être en contact avec le milieu ambiant.

La présente invention concerne des perfectionnements apportés aux dispositifs de ce genre.

Un premier objet de l'invention est de réaliser un corps constituant la source de courant électrique propre à provoquer l'excitation des glandes salivaires qui puisse être conservé de façon confortable et commode à l'intérieur de la cavité buccale en étant commodément maintenu en place sans gêner par sa présence le sujet, tout en permettant un déplacement régulier par intermittence de façon à irriguer de son effet bénéfique l'ensemble des glandes salivaires.

Un autre objet de l'invention est de prévoir un dispositif de ce type qui permette une optimisation et un effet maximum par la disposition au contact des muqueuses intéressées de pôles présentant un dimensionnement assurant la mise en place d'une interface provoquant ainsi un effet de surface maximum.

On peut ainsi obtenir un résultat rapide et une excitation avec un temps de réponse limité de sorte que la pastille peut être conservée dans la bouche pendant une période modérée.

Un mode de réalisation de l'invention vise également à permettre des modulations et des ajustements dans les effets électrolytiques produits par la mise en place de la dragée dans le milieu conducteur constitué par la cavité buccale proprement humectée.

Ainsi qu'on le verra aux termes du présent mémoire descriptif, il est en effet souhaitable de permettre parfois un ajustement des caractéristiques du courant dont la tension s'exerce aux deux pôles opposés de la dragée ; les caractéristiques du courant pouvant répondre à des besoins spécifiques et particuliers, aux seuils de réponse des différents sujets, comme aux différents degrés de disfonctionnement des organes atteints, comme à d'autres particularités.

Et ce mode de réalisation de l'invention permettra de réaliser, à partir d'une structure unique d'appareils, des dispositifs présentant des particularités spécifiques permettant ainsi au prescripteur d'ajuster, en fonction des caractéristiques particulières qui auront été préalablement diagnostiquées, les données et les spécifications des courants propres à assurer la correction du mauvais fonctionnement des glandes salivaires.

Enfin un mode de réalisation de l'invention vise à réaliser dans des conditions particulièrement sûres un dispositif susceptible d'être conservé dans la bouche du sujet sans risque de contamination ou de développement de germes par la mise en oeuvre de matériaux inertes à compatibilité alimentaire, et par une structure totalement fermée et évitant toute immobilisation ou dépôt de débris ou particules susceptibles de constituer une source de développement microbien.

La mise en oeuvre d'une pastille salivaire selon les données de l'art antérieur, telles qu'elles sont notamment exposées dans le brevet français FR-A-2439592, ont posé des problèmes pratiques de réalisation notamment en ce qui concerne les électrodes.

Il est en effet apparu que les électrodes débitant un courant de caractéristiques déterminées au sein d'un milieu électrolytique constituaient de ce fait respectivement une anode et une cathode entraînant par conséquent une destruction progressive de l'anode et l'apparition de dépôt parasite sur la cathode.

L'utilisation de métaux tels que l'acier inoxydable n'avait pas permis de contrôler complètement ce phénomène et de plus l'utilisation d'électrodes en acier inoxydable entraînait une modification importante des caractéristiques du courant en raison de la conductibilité modérément satisfaisante du matériau.

Il était donc nécessaire pour compenser les pertes de surdimensionner les disques afin d'accroître la surface de contact ou encore la capacité de débit de la pile, toutes opérations qui aboutissaient à un appareil, c'est-à-dire une pastille dont la présence dans la cavité buccale devenait une source d'encombrement.

Les inventeurs ont constaté de façon surprenante que l'or, connu de façon traditionnelle pour son caractère inaltérable, permettait non seulement de résister aux sources de corrosion liées au phénomène électrochimique, mais surtout grâce à l'utilisation de ses propriétés de conductibilité élevée, permettait, tout en conservant une pile de faible dimensionnement, de faire transister jusqu'au niveau des muqueuses un courant dont les caractéristiques d'intensité et de tension étaient adaptées au résultat recherché.

Et si l'or a été couramment utilisé notamment dans les prothèses osseuses ou dentaires, pour ses propriétés d'inaltérabilité et de résistance, il n'avait pas été utilisé pour ses propriétés parallèles de conductibilité électrique pour réaliser une électrode insérée dans le milieu vivant afin d'y apporter une source d'excitation dans des conditions évitant toute perte diélectrique liée à un matériau présentant des conditions de résistivité inappropriée.

Et l'utilisation d'électrodes selon l'invention permet la réalisation d'un appareil d'excitation salivaire de dimension réduite apportant au sujet une source d'excitation exactement appropriée aux besoins sans surdimensionnement de l'appareil. L'invention permet en outre de limiter la durée d'utilisation grâce à une excitation efficace. Et pareillement les électrodes échappant à toute corrosion ne perdent pas avec le temps propriétés d'une excellente conductibilité tout en permettant un aspect parfait et hygiénique de la pastille.

A cet effet l'invention concerne un appareil pour l'excitation de la sécrétion salivaire de forme permettant sa mise en place dans la cavité buccale et constitué d'une dragée en matière non conductrice comportant sur chacune de deux faces opposées une électrode formée d'un disque en matériau conducteur, chaque disque étant relié à un des deux pôles d'une pile débitant un courant continu et noyée dans un logement hermétique au sein de ladite dragée ; chaque disque est réalisé en matière synthétique moulée et comporte un revêtement conducteur constitué d'un dépôt d'or sur une épaisseur d'au moins deux microns.

D'autres caractéristiques et avantages de l'invention ressortiront de la description qui suit et qui est donnée en rapport avec une forme de réalisation particulière présentée à titre d'exemple et en se référant aux dessins annexés.

La figure 1 représente une vue générale en perspective de la dragée réalisée conformément à l'invention tandis que :

La figure 2 représente une vue éclatée de la dragée montrant les deux demi-coquilles la composant en position séparée.

La figure 3 représente une vue en coupe longitudinale de la dragée assemblée.

Selon l'ensemble des figures en voit que la dragée 1 est prévue sous forme d'un corps parallélépipèdique applati offrant deux grandes faces respectivement 2 et 2' opposées, les angles du corps parallélépipèdique étant fortement arrondis.

Sur chacune des parois 2, 2' correspondant aux deux grandes faces opposées du parallélépipède aux angles arrondis, est disposé un disque 3, 3' venant affleurer rigoureusement au niveau des faces correspondantes sans discontinuité.

A cet effet les bords des disques d'une part et les bords des logements récepteurs prévus dans chaque face d'autre part, sont usinés à angles vifs et parfaitement ébavurés de façon à assurer une insertion rigoureuse du disque dans son logement tandis que la paroi supérieure du disque est rigoureusement coplanaire avec la paroi du reste de la dragée sans discontinuité.

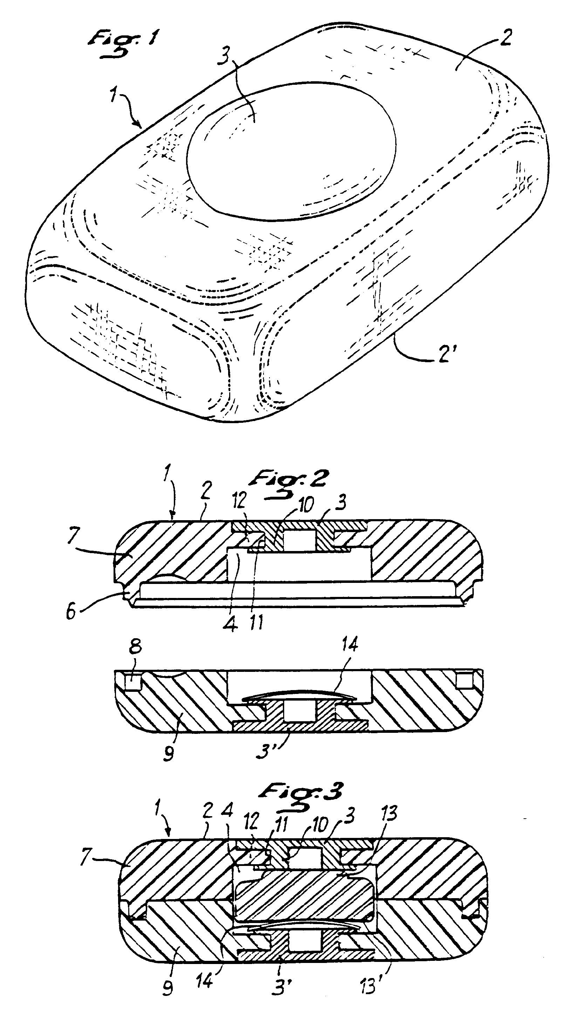

Comme on le voit sur la figure 2 le logement prévu sur chacune des parois 2, 2' de la dragée traverse cette paroi pour déboucher jusqu'à une capacité intérieure 4.

A cet effet la dragée est constituée de deux moitiés séparées selon un plan médian parallèle aux deux grandes faces 2, 2' et chaque moitié est constituée sous forme d'une demi-coquille disposée en regard de l'autre ; les deux demi-coquilles sont solidarisées par un plan de joints correspondant au plan médian parallèle aux deux faces 2, 2'.

La solidarisation des deux demi-coquilles est obtenue par engagement d'une nervure 6 périphérique prévue sur une des deux demi-coquilles 7 et engagée dans la rainure 8 prévue sur la demi-coquille opposée 9.

La nervure 6 présente une extrémité biseautée et angulaire venant à engagement dans le fond de la rainure 8 et permettant une solidarisation de la rainure dans sa nervure réceptrice par soudure aux ultrasons. On obtient ainsi une solidarisation parfaite des deux demi-coquilles et une étanchéité totale de la chambre ou capacité intérieure 4.

Par ailleurs le disque 3, 3' est prolongé vers l'intérieur de la dragée, par un manchon cylindrique 10 pourvu d'une gorge circulaire 11 réceptrice de la collerette 12 venue du bord du logement permettant l'insertion du disque 3. Dans ces conditions la mise en place et la solidarisation du disque 3 refermant l'ouverture constituant son logement sont assurées dans des conditions particulièrement stables et hermétiques.

La dragée elle-même est réalisée en matériau synthétique à compatibilité alimentaire et possédant des propriétés de rigidité et de résistance voulues ; plus spécialement la dragée est réalisée en métacrylate comportant éventuellement un colorant à compatibilité alimentaire, tandis que le disque 3 est réalisé en matière synthétique avec revêtement or.

La capacité intérieure 4 totalement étanche dans les conditions prévues ci-dessus reçoit, avant soudure des deux demi-coquilles, une pile électrique de préférence de modèle standard (la capacité 4 étant prévue avec des dimensions voulues à cet effet pour recevoir exactement la pile standard).

La pile est prévue avec deux pôles opposés respectivement 13, 13' venant en contact de chacun des disques 3 par la face arrière du manchon cylindrique 10 ; et pour assurer un contact permanent sous tension avec un rattrapage de jeu éventuel, l'une des faces, par exemple la grande face 13′ de la pile est en contact avec la face arrière correspondante du disque 3′ par l'intermédiaire d'un organe ressort ; cet organe peut être avantageusement constitué d'une rondelle en matériau élastiquement déformable et légèrement pliée, la déformation élastique de cette rondelle 14 assurant d'une part le contact électrique et d'autre part le maintien sous légère tension mécanique de la pile entre les deux disques 3, 3′.

Selon une variante de réalisation de l'invention on prévoit d'insérer entre un des pôles ou bornes de la pile et un disque constituant une des deux électrodes de la dragée, une pastille en matériau diélectrique comportant des propriétés de capacité ou de résistivité voulue et propre à assurer la modulation ou une modification appropriée des caractéristiques du courant continu acheminé depuis la pile aux faces 3, 3′ des disques constituant les électrodes de la dragée.

Il est donc possible dans ces conditions, grâce à la mise en place d'une interface comportant des capacités ou des propriétés électriques appropriées d'aménager ou de moduler les caractéristiques du courant de façon à réaliser, à partir des mêmes éléments standard et sans modification des autres pièces, des dragées comportant des caractéristiques de tension électrique appropriée et adaptée à chaque cas.

Les piles du commerce utilisables pour cette application débitant un courant de tension sensiblement égale à 1,58 volt, l'insertion de la résistance (qui pourra prendre la place de la rondelle métallique 14) permettrait de moduler la tension et de l'abaisser à un niveau compris entre 1,58 et 1,2 volt (cette dernière valeur correspondant au seuil inférieur d'efficacité) ; on pourrait ainsi offrir des dragées calibrées à 1,40 ou 1,30 volts correspondant à différents cas de malade.

Et selon une variante on peut également disposer en série deux ou plus piles extra plates superposées en assurant ainsi une multiplication par un nombre n correspondant au nombre de piles, de la tension apportée aux disques 3, 3′ constituant les électrodes de la dragée.

On comprend que la forme spécifique de la dragée permette son maintien et sa conservation en place à l'intérieur de la cavité buccale, par exemple sous la langue sans gêner en aucune façon le sujet pour un orateur et certaines disciplines sportives à l'exception de la natation, la dragée selon l'invention pourra être placée dans le triangle rétro-molaires, c'est-à-dire entre les dernières molaires du maxillaire inférieur et la joue ; ainsi l'élocution reste bonne, et la langue peut transposer la dragée facilement du côté gauche au côté droit et réciproquement sans la laisser stationner trop longtemps ; cette caractéristique est particulièrement importante dans le cas d'orateurs ayant besoin par conséquent d'une source de salive propre à faciliter l'élocution sans cependant que cette dernière soit gênée par la présence d'un corps étranger.

Ainsi la pastille peut facilement être déplacée par le travail de la langue de façon à apporter la source d'excitation aux différents endroits successivement où elle doit produire ses effets ; l'excitation engendrée étant ainsi promenée dans les différentes zones appropriées de la capacité buccale en multipliant géographiquement les effets de production salivaire.

Ce déplacement est rendu commode par la forme plate qui, sans gêner ou handicaper le mouvement de la langue permet de contrôler plus facilement le déplacement de la dragée.

L'absence d'angles permet en même temps d'éviter tout contact agressif.

Les surfaces parfaitement polies de la résine (métacrylate) et des disques métalliques ainsi que la parfaite continuité assurée en toute zône de la paroi de la dragée évite tout dépôt de matière ou insertion de débris ou stagnation de matières organiques susceptibles de constituer le siège de la prolifération de germes.

Ceci facilite grandement l'hygiène et le maintien de la dragée dans des conditions d'aseptie parfaite.

La forme plate de la dragée permettant le contrôle au sein de la bouche évite également toute déglutition accidentelle.

Cependant au cas même où une telle déglutition viendrait à se produire, par suite d'un mouvement inopiné, il suit que les formes arrondies et fuyantes données au volume de la dragée permettent son déplacement avec le bol alimentaire au sein du système digestif et son cheminement dans le tractus intestinal sans risque de lésion ou de dommages aux parois des tubes ou cavités traversés, la dragée se retrouvant finalement évacuée naturellement et ainsi finalement récupérable (au prix d'un simple nettoyage).

Suivant l'intensité de l'utilisation la dragée pourra être efficace pendant une durée de plusieurs mois allant jusqu'à un an ; la pile étant alors épuisée, l'ensemble peut être jeté. La durée d'efficacité dépend non seulement du temps d'utilisation mais aussi du degré d'acidité de la salive ; cette durée est de l'ordre de 6 à 8 mois en général ; à noter quand la tension mesurée au voltmètre tombe à un niveau de l'ordre de 1 à 1,2 volt, l'effet de stimulation cesse pratiquement.

La réalisation de la dragée selon l'invention permet en effet de réaliser en série les dragées du type jetable mais à usage prolongé, les modalités de réalisation permettant une fabrication en série et à faible coût.

De préférence selon l'invention chaque disque 3, 3′ est en matériau synthétique tel que ABS, méthacrylate ou PVC et comporte un revêtement d'or 24 carats sur une épaisseur de 2 microns.

Selon une variante la pastille est constituée d'un bloc monopièce en matière synthétique et obtenue par injection moulage et contenant sous forme d'insert une pile dont chaque pôle, supérieur et inférieur, est en contact avec un des deux disques, chaque disque affleurant au niveau de la surface du bloc synthétique.

## Revendications

1. Appareil pour l'excitation de la sécrétion salivaire, de forme permettant sa mise en place dans la cavité buccale et constitué d'une dragée en matière non conductrice comportant sur chacune de deux faces opposées une électrode formée d'un disque en matériau conducteur, chaque disque (3, 3') étant relié à un des deux pôles d'une pile débitant un courant continu et noyée dans un logement (4) hermétique au sein de ladite dragée, caractérisé en ce que chaque disque est réalisé en matière synthétique moulée et comporte un revêtement conducteur constitué d'un dépôt d'or sur une épaisseur d'au moins deux microns.

2. Appareil selon la revendication 1, caractérisé en ce que la dragée est constitué d'un bloc monopièce en matière synthétique obtenu par injection moulage et contenant sous forme d'insert l'ensemble galvanique constitué de la pile, en contact par chacun de ses pôles avec un disque affleurant au niveau de la surface du bloc synthétique.

3. Appareil selon la revendication 1, caractérisé en ce que la dragée est réalisée à partir de deux demi-coquilles en matière synthétique moulée et séparées par un plan de joint médian, chaque demi-coquille (7-9) comportant des nervures (6) et rainures (8) de solidarisation venant en regard d'une demi-coquille sur l'autre, l'espace intérieur constituant un logement (4) récepteur de la pile d'alimentation, et chaque disque (3, 3') constituant chacun une des deux électrodes, traverse la paroi de la demi-coquille constituant la moitié correspondante de la dragée et il est à cet effet inséré dans un logement de profil convenable prévu dans cette demi-coquille, le disque étant prolongé vers l'intérieur de la dragée par un manchon (10) cylindrique pourvu d'une gorge (11) réceptrice d'une collerette (12) prévue sur le bord coaxial et situé en regard dudit logement, l'engagement de la collerette (12) dans ladite gorge (11) assurant la solidarisation et le positionnement ferme et hermétique du disque constituant l'électrode sur la face correspondante de la dragée, et la pile située dans le logement (4) intérieur comporte sur au moins un de ses pôles, constitué de préférence par le fond le plus large de ladite pile, un organe ressort formé par une rondelle métallique (13) pourvue d'un léger repli assurant le maintien sous tension de chacun des deux pôles de la pile par chacun des deux disques (3, 3') opposés.

4. Appareil selon l'une des revendications 1, 2 ou 3, caractérisé en ce que il comporte intérieurement un organe di-électrique de résistance convenable inséré entre un des pôles de la pile et l'un des disques, et apte à ajuster les caractéristiques du courant continu dont la tension s'exerce sur les deux disques opposés de la dragée.

## Patentansprüche

1. Gerät zur Anregung der Speichelsekretion, dessen Form das Einlegen in die Mundhöhle ermöglicht und das aus einer Pastille aus nicht leitendem Material besteht, die auf jeder von zwei entgegengesetzten Seiten eine Elektrode in Form einer Scheibe aus leitendem Material aufweist, wobei jede Scheibe (3, 3') mit einem von zwei Polen einer Batterie verbunden ist, die einen Gleichstrom abgibt und in einen hermetisch abgeschlossenen Sitz (4) innerhalb der Pastille eingebettet ist, dadurch gekennzeichnet, daß jede Scheibe aus einem geformten Kunststoffmaterial besteht und eine leitende Beschichtung aufweist, die aus einer Goldabscheidung mit einer Dicke von mindestens zwei μm besteht.

2. Gerät nach Anspruch 1, dadurch gekennzeichnet, daß die Pastille aus einem einstückigen Block aus Kunststoffmaterial besteht, der durch Spritzguß erhalten wurde und die aus der Batterie bestehende galvanische Anordnung in Form einer Einlage enthält, wobei die Batterie durch jeden ihrer Pole mit einer bis zur Höhe der Oberfläche des Kunststoffblocks reichenden Scheibe in Berührung steht.

3. Gerät nach Anspruch 1, dadurch gekennzeichnet, daß die Pastille aus zwei Halbschalen aus geformtem Kunststoffmaterial hergestellt ist, die durch eine mittlere Dichtungsebene getrennt sind, wobei jede Halbschale (7-9) Rippen (6) und Nuten (8) zur festen Verbindung aufweist, die sich von einer zur anderen Halbschale gegenüberliegen, wobei der Innenraum einen Sitz (4) zur Aufnahme der Versorgungsbatterie bildet und jede Scheibe (3, 3') jeweils eine der zwei Elektroden bildet und die Wand der Halbschale durchsetzt, welche die entsprechende Hälfte der Pastille bildet, wobei sie zu diesem Zweck in einen in dieser Halbschale vorgesehenen Sitz von geeignetem Profil eingesetzt ist und jede Scheibe zum Inneren der Pastille hin durch eine zylindrische Manschette (10) verlängert ist, die mit einer Nut (11) zur Aufnahme eines Kragens (12) versehen ist, der am koaxialen Rand vorgesehen und dem Sitz gegenüber angeordnet ist, wobei der Eingriff des Kragens (12) in die Nut (11) die feste Verbindung und die sicher und hermetisch abgeschlossene Anordnung der Scheibe gewährleistet, welche die Elektrode auf der entsprechenden Seite der Pastille bildet, und die im inneren Sitz (4) angeordnete Batterie auf mindestens einem ihrer Pole, der vorzugsweise vom größeren Boden der Batterie gebildet ist, ein Federelement aufweist, das von einer Federscheibe (13) aus Metall gebildet ist, die mit einer leichten Biegung versehen ist, welche gewährleistet, daß jeder der zwei Pole der Batterie durch jede der zwei entgegengesetzten Scheiben (3, 3') unter Spannung gehalten ist.

4. Gerät nach einem der Ansprüche 1, 2 oder 3, dadurch gekennzeichnet, daß es innen ein dielektrisches Element von geeignetem Widerstand aufweist, das zwischen einen der Pole der Batterie und eine der Scheiben eingesetzt ist und dazu dient, die Charakteristika des Gleichstroms einzustellen, wobei das Element die beiden entgegengesetzten Scheiben der Pastille unter Spannung hält.

stituted by the wider bottom of said battery, a spring member formed by a metal washer 13 provided with a slight fold ensuring maintaining under voltage of each of the two poles of the battery by each of the two opposite discs 3, 3'.

4. Apparatus according to one of Claims 1, 2 or 3, characterized in that it comprises, internally, a suitable dielectric resistance member inserted between one of the poles of the battery and one of the discs, and adapted to adjust the characteristics of the direct current whose voltage is exerted on the two opposite discs of the pellet.

## Claims

1. Apparatus for stimulating the secretion of saliva, of form allowing it to be placed in the mouth, and constituted by a pellet made of non-conductive material comprising on each of two opposite faces an electrode formed by a disc of conductive material, each disc 3, 3' being connected to one of the two poles of a battery supplying a direct current and embedded in a hermetic housing 4 within said pellet, characterized in that each disc is made of moulded synthetic material and comprises a conductive coating constituted by a deposit of gold over a thickness of at least two microns.

2. Apparatus according to Claim 1, characterized in that the pellet is constituted by a one-piece block of synthetic material obtained by injection moulding and containing in the form of insert the galvanic assembly constituted by the battery, in contact by each of its poles with a disc flush with the level of the surface of the synthetic block.

3. Apparatus according to Claim 1, characterized in that the pellet is made from two half-shells of moulded synthetic material, separated by a median mould join, each half-shell 7-9 comprising ribs 6 and grooves 8 for connection opposite one another from one half-shell to the other, the inner space constituting a housing 4 receiving the supply battery, and each disc 3, 3' each constituting one of the two electrodes, passes through the wall of the half-shell constituting the corresponding half of the pellet and it is to that end inserted in a housing of suitable section provided in this half-shell, the disc being extended inwardly of the pellet by a cylindrical bush 10 provided with a groove 11 receiving a flange 12 provided on the coaxial edge and located opposite said housing, the engagement of the flange 12 in said groove 11 ensuring connection and firm and hermetic positioning of the disc constituting the electrode on the corresponding face of the pellet, and the battery located in the inner housing 4 comprises on at least one of its poles, preferably con-

*Fig. 1*

*Fig. 2*

*Fig. 3*